**Europäisches Patentamt**

**European Patent Office**

**Office européen des brevets**

(11) Publication number : **0 514 130 A2**

(12) # EUROPEAN PATENT APPLICATION

(21) Application number : **92304260.0**

(22) Date of filing : **12.05.92**

(51) Int. Cl.⁵ : **A61K 37/02**

(30) Priority : **13.05.91 US 699092**

(43) Date of publication of application :
**19.11.92 Bulletin 92/47**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(71) Applicant : **CURATORS OF THE UNIVERSITY**
**OF MISSOURI**
**509 Lewis Hall, University of Missouri**
**Columbia, MO 65211 (US)**

(72) Inventor : **Sato, Masahiko**
**10 Windsor Court**
**Lansdale, PA 19446 (US)**
Inventor : **Gorski, Jeffrey P.**
**3900 West 91st Street**
**Prairie Village, Kansas (US)**
Inventor : **Rodan, Gideon A.**
**827 Deerfield Lane**
**Bryn Mawr, PA 19010 (US)**

(74) Representative : **Thompson, John Dr. et al**
**Merck & Co., Inc. European Patent**
**Department Terlings Park Eastwick Road**
**Harlow, Essex CM20 2QR (GB)**

(54) **Bone acidic glycoprotein (BAG-75) for inhibiting bone resorption.**

(57)    Disclosed is a new method for treating bone disease in mammals, which is associated with increased bone resorption activity (dissolution or breakdown), and involves the new use and administration of known bone acidic glycoproteins 75 (BAG 75), having a molecular weight of 75kDa as determined by SDS-PAGE, or the 50kDa (SDS-PAGE) fragment thereof, wherein BAG 75 contains an N-terminal sequence of :

```
       1              5              10
  Leu-Pro-Val-Ala-Arg-Tyr-Gln-Asn-Thr-Glu-Glu-Glu-Glu,
```

(SEQ. ID NO. 1), (LPVARYQNTEEEE). The method is applicable and indicated for osteoporosis, Paget's disease, hyperparathyroidism, hypercalcemia of malignancy, osteopenia due to bone metastases, bone loss due to immobilization, sex hormone deficiency, glucocorticoid treatment, periodontal disease, and periarticular erosion in rheumatoid arthritis.

EP 0 514 130 A2

FIG. 1

EP 0 514 130 A2

## BACKGROUND OF THE INVENTION

### 1. Field of the Invention

This invention relates to a new method for treating mammals suffering from bone disease associated with increased bone resorption involving the use of a therapeutically effective amount of bone acidic glycoprotein 75, or a 50kDa fragment thereof, containing an N-terminal sequence of:

$$1 \qquad\qquad\qquad\qquad\qquad\qquad 10$$

Leu-Pro-Val-Ala-Arg-Tyr-Gln-Asn-Thr-Glu-Glu-Glu-Glu,

(SEQ ID NO. 1).

### 2. Brief Description of Disclosure in the Art

The current major bone diseases of public concern are osteoporosis, hypercalcemia of malignancy, osteopenia due to bone metastases, periodontal disease, hyperparathyroidism, periarticular erosions in rheumatoid arthritis, Paget's disease, immobilization-induced osteopenia, and glucocorticoid treatment.

All these conditions are characterized by bone loss, resulting from an imbalance between bone resorption (breakdown) and bone formation, which continues throughout life at the rate of about 14% per year on the average. However, the rate of bone turnover differs from site to site, for example it is higher in the trabecular bone of the vertebrae and the alveolar bone in the jaws than in the cortices of the long bones. The potential for bone loss is directly related to turnover and can amount to over 5% per year in vertebrae immediately following menopause, a condition which leads to increased fracture risk.

There are currently 20 million people with detectable fractures of the vertebrae due to osteoporosis in the U.S. In addition, there are 250,000 hip fractures per year attributed to osteoporosis, which are associated with a 12% mortality rate within the first two years and 30% of the patients require nursing home care after the fracture.

All the conditions listed above would benefit from treatment with agents which inhibit bone resorption.

Bone acidic glycoprotein 75 (BAG 75) is a new 75kDa naturally occuring glycoprotein known in the art and its properties and preparation are described by J.P. Gorski et al. in J. Biological Chemistry, vol. 265, pp. 14956-14963 (1990) and ibid., vol. 263, pp. 15938-15945 (1988). These articles describe that BAG 75 is a phosphorylated glycoprotein from the mineralized compartment of rat calvarial (skull) tissue with a limited homology to the matrix protein osteopontin (2ar, pp69, Sppl, OP) , is antigenically distinct, predominantly localized to calcified tissues, and represents a major product of osteoblasts (cells involved with bone formation) and may be able to undergo a characteristic fragmentation in vivo and in vitro.

However, there is no description or suggestion of the effect of BAG 75 on the inhibition of osteoclasts (cells involved with bone dissolution) in bone resorption.

## SUMMARY OF THE INVENTION

It has been found that BAG 75 (purified from bone) is effective in complexing with mammalian bone to inhibit the action of osteoclasts in causing bone resorption (dissolution). The inhibition is primarily caused by the formation of a BAG 75/bone complex rather than inhibitions between osteoclasts and free BAG 75 in the biological media.

Also, it has been found that a 50kDa fragment (as measured by SDS-PAGE) of BAG 75, containing the N-terminal sequence of:

$$1 \qquad\qquad 5$$

Leu-Pro-Val-Ala-Arg-Tyr, (SEQ ID NO. 2)

is also effective in inhibiting bone resorption by osteoclasts.

It has been shown for the first time, that: BAG 75 and the 50kDa fragment are potent inhibitors of bone resorption; BAG 75 impedes (modulates) osteoclastic interaction with bone; it is the BAG 75/bone complex that inhibits bone resorption, not interaction between osteoclasts and BAG 75 free in the biological media; and because BAG 75 is a natural bone protein, it can have important physiological advantages over synthetic inhibitors of bone resorption.

By this invention there is provided a process for treating mammals suffering from a bone condition caused or mediated by increased bone resorption, who are in need of such treatment, comprising the step of administering to said mammals a therapeutically effective amount of bone acidic glycoprotein 75, containing the N-terminal sequence of:

```
          1                      5                          10
        Leu-Pro-Val-Ala-Arg-Tyr-Gln-Asn-Thr-Glu-Glu-Glu-Glu,
```

(SEQ ID NO. 1), or a 50kDa fragment thereof, or a smaller form, exhibiting this biological activity derived from BAG 75, which results in decreased bone resorption in the mammal.

## BRIEF DESCRIPTION OF THE DRAWINGS

Figure 1 is a graph of the effect of various non-collagenous bone proteins, including osteopontin, BAG 75, vitronectin, fibronectin, the 50 kDa fragment of BAG 75 (50 BAG) and bone sialoprotein (SpII, BSP) on the bone resorption activity of isolated chicken osteoclasts.

Figure 2 is a graph of the effect of BAG 75 and osteopontin, a bone matrix protein, on the bone resorption activity of rat osteoclasts.

Figure 3 is a graph showing the kinetics of bone interactions in the presence and absence of BAG 75 or osteopontin with chicken osteoclasts.

## BRIEF DESCRIPTION OF THE INVENTION AND PREFERRED EMBODIMENTS

Mammalian bone is composed of cells, hydroxyapatite, collagen, blood vessels, nerves and non-collagenous matrix proteins.

Most of these non-collagenous proteins, which include osteopontin, bone sialoprotein, osteocalcin and osteonectin, have been shown to be involved in the osteoblastic mediated processes for bone formation.

The recently isolated BAG 75 glycoprotein by Gorski, et al., (supra) has been unexpectedly found to be involved in the osteoclastic mediated process of bone resorption.

BAG 75 was extracted from rat calvaria and purified by ion exchange and hydroxyapatite chromatographic steps. As reported in the literature, the protein has a molecular weight of 75,000, i.e. 75 kDa, as determined by SDS-PAGE, contains 29.32 molar content of acidic amino acid residues, a 7.0% (w/w) content of sialic acid and a 7.9% molar content of organic phosphate.

It has an N-terminal sequence of:

```
          1                                            10
        Leu-Pro-Val-Ala-Arg-Tyr-Gln-Asn-Thr-Glu-Glu-Glu-Glu,
```

(SEQ ID NO. 1). The N-terminal sequence does not show any sequence homology with the primary structure of human bone sialoprotein and does not bind anti-bone sialoprotein antibodies. The N-terminal sequence of bone acidic glycoprotein-75 is unique, however, and a 33% homology is shared with the rat matrix protein osteopontin over the first 15 residues. Some affinity-purified antibodies against osteopontin can cross-react to bone acidic glycoprotein-75 and to bone sialoprotein upon immunoblotting, however this result is likely due to impurities of BAG-75 and bone sialoprotein in osteopontin preparations used for immunization and to prepare immunoaffinity adsorbents. Antibodies directed against N-terminal residues 3-13 of BAG-75 or against the protein as a whole are specific and do not crossreact with bone sialoprotein, osteopontin, or other proteins from bone whether as purified proteins or as components of crude calvarial extracts. However, bone acidic glycoprotein-75 is a new phosphorylated glycoprotein from the mineralized compartment of rat calvarial tissue with a limited structural homology to osteopontin.

The known 50kDa fragment, described above by Gorski, et al., can be formed by splitting the 75kDa glycoprotein by long-term storage or by proteolysis with stromelysin.

BAG 75 or the 50kDa glycoprotein are used to inhibit the conditions of bone resorption in mammals needing such treatment, i.e. humans suffering from osteoporosis, Paget's disease, hypercalcemia of malignancy, and the like.

The glycoprotein can be delivered to the mammal via a conventional injectable formulation, i.e. saline solution, by a once-a-day dosage regimen, in an amount of about 50 nanomolar per day per person (average 70 Kg weight).

The following examples are illustrative of the invention and should not be used to limit the spirit or scope of the invention as claimed.

## EXAMPLE 1

BAG 75 purified from rat calvaria (skulls) can be obtained as described in the articles above by J. Gorski (Univ. Missouri). The 50kDa fragment can also be obtained from BAG 75 by fragmentation, i.e. with stromelysin, as described by Gorski above.

The chicken osteoclast bone resorption assay is described as follows:

## Blair - Zambonin-Zallone Chicken Osteoclast Model

Osteoclasts were isolated from chickens by methods described in Blair et al. (J. Cell Biol 102:1164, 1986). Medullary bone was harvested from split femora and tibiae of Dekalb XL laying hens maintained on a $Ca^{++}$ deficient diet (5070C-9, Purina Mills Inc., St. Louis, MO) for 2-4 wk. The bone suspension was washed in PBS at 4°C, pressed through a 110 μm nylon mesh, incubated in 0.2% NaCl for 2 minutes at 37°C to lyse red blood cells, and then sedimented through a 70% serum gradient for 90 minutes at 4°C. The bottom 5 ml was resuspended over a 3 step Nycodenz gradient (1.073, 1.099, 1.143 $g/cm^3$) (Accurate Chemical & Scientific Corp., Westbury, NY) and centrifuged at 350 g for 20 minutes at 4°C. Cells from below the first band to above the pellet were pooled and resuspended into alpha-MEM, pH 7.0-7.2, 10% fetal calf serum, antibiotics (Gibco Laboratories, Grand Island, NY) plus 2-5 μg/ml cytosine-1-B-D-arabinofuranoside at 4°C. The cell suspension was aliquoted into Costar plates (Cambridge, MA) to yield 1000-4000 $osteoclasts/cm^2$.

Bone resorption was assayed by measuring the [³H] released from labelled bone particles aliquoted at 100 $μg/cm^2$ onto chicken osteoclasts in 48 well plates (Costar Corp.). Bone particles (20-53 μm) were made by seiving crushed bone from rats injected with [³H] proline (Amersham Corp., Arlington Hts., IL) by the known art procedure as described by Blair, et al. J. Cell Biol. 102:1164 (1986). [³H] released into the media was quantitated with a liquid scintillation counter (LKB Instruments, Inc., Gaitherburg, MD). Resorption was typically assayed between days 4-6 in culture.

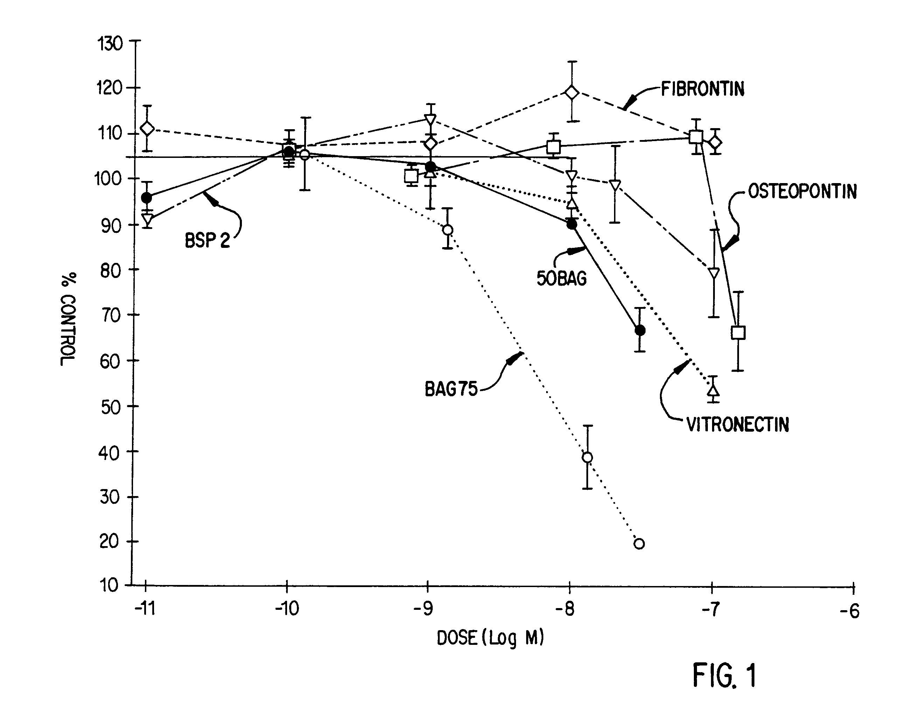

Figure 1 shows the resulting effects of rat bone acidic glycoprotein 75 (BAG 75) on the bone resorption activity of isolated chicken osteoclasts. For comparison, the effect of rat BAG 75 on resorption was plotted with the effect of other noncollagenous bone proteins on resorption, including rat osteopontin, human vitronectin, human fibronectin, rat bone sialoprotein, and the 50kDa fragment of rat BAG 75, as determined by this same assay. The reason for comparing BAG 75 to osteopontin, vitronectin, fibronectin, and BSP2 was to examine the possibility that BAG 75, as well as the others, may mediate cell interactions to bone, which is clearly affirmed by the data. Resorption is expressed as percent control resorption in the absence of added factors, with 100% equal to 16- 39 μg bone (mean±SEM, n= 3-6). As seen from Figure 1, BAG 75 was significantly more effective than the other proteins in inhibiting resorption with an $IC_{50}$ = 10 nM. The second most effective inhibitor was the 50kDa fragment with an $IC_{50}$=50 nM.

## EXAMPLE 2

Boyde-Chamber-Dempster Rat

## Osteoclast Bone resorption Model

This osteoclast model system and methodology is described extensively by Sato and Grasser (J. Bone Min. Res. 5:31, 1990) and Arnett and Dempster (Endocrinology 120:602, 1987). Briefly, cells isolated from the long bones of 1-3 day old rats (Sprague-Dawley) were aliquoted onto 4.4 mm x 4.4 mm x 0.2 mm bone slices from the diaphysis of bovine femur. After overnight incubation, resorption was assayed by removing cells, fixing bone slices, staining with toluidine blue and quantitating resorption pits by reflected polarized light microscopy as detailed in the methodology of the above reference of Sato and Grasser (1990).

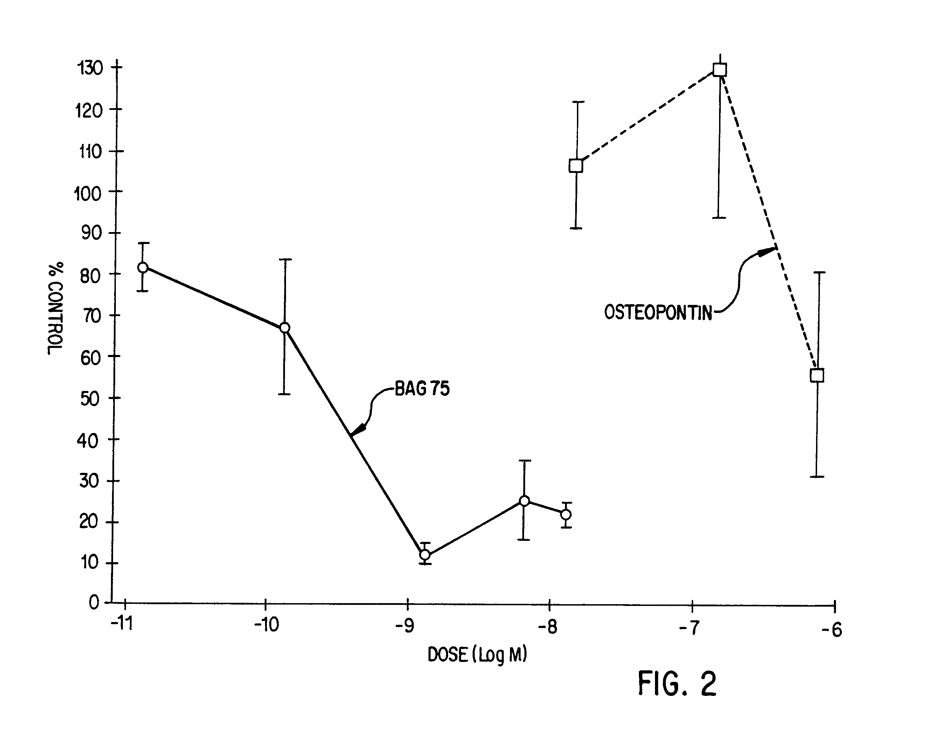

Figure 2 illustrating the data shows that BAG 75 potently inhibits the resorption activity of isolated rat osteoclasts with $IC_{50}$=1 nM, which was three orders of magnitude below the resorption $IC_{50}$ for osteopontin. Data are expressed as percent control resorption in the absence of added factors, with 100% equal to 23- 289 resorption lacunae/bone slice (mean±SEM, n= 6-8).

EXAMPLE 3

Attachment Assay

Attachment of bone to chicken osteoclasts in 48 well plates (Costar Corp.) was quantitated as a function of time after addition of 100 $\mu$g/well of bone particles (20-53 $\mu$m) prelabelled with [$^3$H] proline. Attachment was monitored in the presence and absence of 10 nM BAG 75 or osteopontin in complete alpha-MEM media. Free bone was defined as particles liberated by 3x swirling of cultures with media, while the bound pool was defined as particles liberated after 4 hour incubation 1 N NaOH which completely disrupted all cells. Samples were then combusted in a Packard 306 oxidizer (Packard Inst., Sterling, VA) and the [$^3$H] counts measured with and LKB scintillation counter.

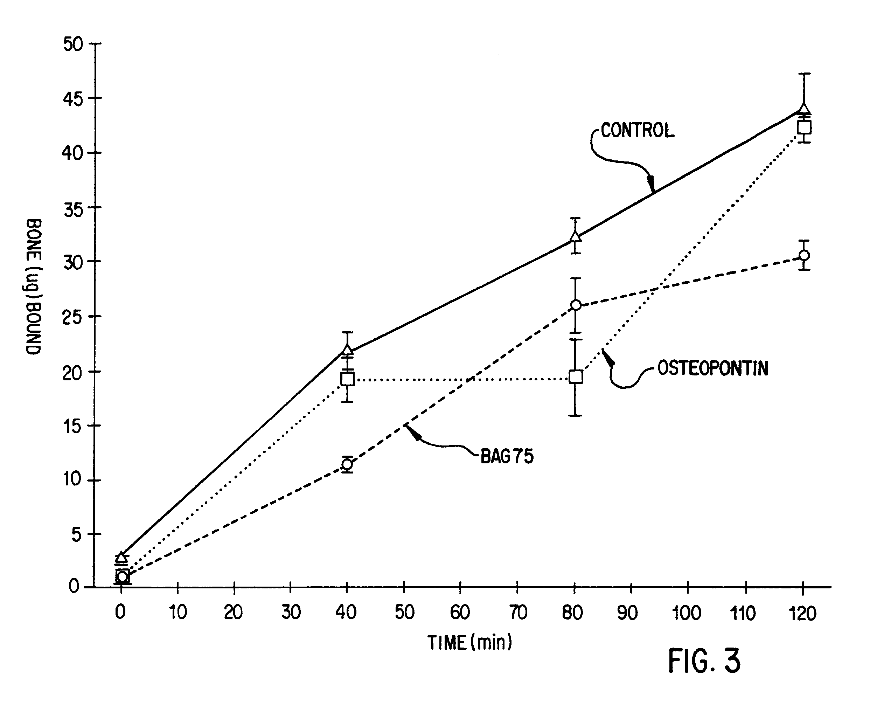

The kinetics of bone interactions with chicken osteoclasts was plotted, as shown in Figure 3. The time dependent association of bone particles was examined in the absence (control) and presence of 10 nM osteopontin or 10 nM BAG 75. It is seen from the data that, following 120 min, BAG 75 appeared to significantly impede the interaction of bone particles to chicken osteoclasts.

EXAMPLE 4

In an effort to ascertain the mechanism for the BAG 75 inhibition, resorption was examined for osteoclasts or bones transiently incubated with BAG 75. Resorption activity was measured for chicken osteoclasts initially incubated with 50 nM BAG 75 (1 hr, 37°C), washed 2x and then incubated with 100ug of [$^3$H] bone particles (20- 53 um). Alternatively, [$^3$H] bone particles were incubated with 50 nM BAG 75 (1hr, 37°C), washed 2x and then fed to chicken osteoclasts. In the last set, the resorption activity was measured for osteoclasts incubated concurrently with 50 nM BAG 75 and [$^3$H] bone particles. Resorption data are presented as percent control with 100%= 44-47 ug bone (mean±SEM, n=3-6).

As seen below in Table I, transient incubation of osteoclasts with BAG 75 had no effect (did not inhibit) resorption. Alternatively, transient incubation of bones with BAG 75 resulted in significant (40%) inhibition of resorption. These data show that it is the BAG 75/bone complex that inhibits osteoclastic activity rather than interactions between osteoclasts and BAG 75 free in the media. These cumulative data are strong evidence to show that BAG 75 is a powerful modulator of osteoclastic activity that may have physiological advantages over synthetic inhibitors of bone resorption.

## TABLE I

| Treatment | BAG 75 | Resorption |
|---|---|---|
| Washed osteoclasts | 50 nM | 100±10.8% |
| Washed bones | 50 nM | 61±5.5% |
| Concurrent | 50 nM | 12±1.1% |

SEQUENCE LISTING

(2)  INFORMATION FOR SEQ ID NO: __1__

  (i)  SEQUENCE CHARACTERISTICS:

    (A)  LENGTH: __13__

    (B)  TYPE: __amino acid__

    (C)  STRANDEDNESS: __single__

    (D)  TOPOLOGY: __linear__

```
1              5                    10         13
Leu Pro Val Ala Arg Tyr Gln Asn Thr Glu Glu Glu Glu
```

(3)  INFORMATION FOR SEQ ID NO: __2__

  (i)  SEQUENCE CHARACTERISTICS:

    (A)  LENGTH: __6__

    (B)  TYPE: __amino acid__

    (C)  STRANDEDNESS: __single__

    (D)  TOPOLOGY: __linear__

```
1              5
Leu Pro Val Ala Arg Tyr
```

## Claims

1. The use of bone acidic glycoprotein 75, or a fragment thereof, which contains the N-terminal sequence of:

```
1                  5                          10          13
Leu-Pro-Val-Ala-Arg-Tyr-Gln-Asn-Thr-Glu-Glu-Glu-Glu,
```

(SEQ ID No. 1), for the manufacture of a medicament for treating mammals suffering from a bone condition caused or mediated by increased bone resorption.

2. The use as claimed in Claim 1 wherein said fragment is a 50kDa fragment, containing an N-terminal sequence:

```
1                  5
Leu-Pro-Val-Ala-Arg-Tyr, (SEQ ID NO. 2).
```

3. The use as claimed in Claim 1 wherein said condition is selected from hypercalcemia of malignancy, Paget's disease, osteopenia due to bone metastases, bone loss due to immobilization, sex hormone deficiency, glucocorticoid treatment, periodontal disease, hyperparathyroidism, periarticular erosion in rheumatoid arthritis, and osteoporosis.

FIG. 1

FIG. 2

EP 0 514 130 A2

FIG. 3